(19) Europäisches Patentamt
European Patent Office
Office européen des brevets

(11) Veröffentlichungsnummer: **0 163 855**
**B1**

# (12) EUROPÄISCHE PATENTSCHRIFT

(45) Veröffentlichungstag der Patentschrift: **21.06.89**

(51) Int. Cl.⁴: **C 07 D 401/06**, C 07 D 213/61, C 07 D 213/38, C 07 D 213/70, A 01 N 43/50, A 01 N 43/54, A 01 N 43/48

(21) Anmeldenummer: **85104254.9**

(22) Anmeldetag: **09.04.85**

## (54) Nitromethylen-Derivate, Zwischenprodukte für diese, Verfahren zu ihrer Herstellung sowie Insektizide.

(30) Priorität: **13.04.84 JP 72966/84**
**29.06.84 JP 132943/84**

(43) Veröffentlichungstag der Anmeldung: **11.12.85 Patentblatt 85/50**

(45) Bekanntmachung des Hinweises auf die Patenterteilung: **21.06.89 Patentblatt 89/25**

(84) Benannte Vertragsstaaten: **AT BE CH DE FR GB IT LI NL SE**

(56) Entgegenhaltungen:
**EP-A-0 099 685**
**DE-A-2 514 402**
**DE-A-2 732 660**
**FR-A-1 511 398**
**GB-A-2 014 147**
**US-A-3 252 860**
**US-A-3 971 774**
**US-A-4 002 765**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Shiokawa, Kozo, 210- 6, Shukugawara Tama- ku, Kawasaki- shi Kanagawa- ken (JP)**
Erfinder: **Tsuboi, Shinichi, 3-26- 1, Hirayama, Hino-shi Tokyo (JP)**
Erfinder: **Kagabu, Shinzo, 432- 131- 105, Terada-machi, Hachioji- shi Tokyo (JP)**
Erfinder: **Moriya, Koichi, 39- 15, Namiki- cho, Hachioji- shi Tokyo (JP)**

(74) Vertreter: **Ernst, Hilmar, Dr., Bayer AG Konzernverwaltung RP Patentabteilung, D-5090 Leverkusen, Bayerwerk (DE)**

**EP 0 163 855 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue Nitromethylen-Derivate, Zwischenprodukte für diese, Verfahren zu ihrer Herstellung sowie Insektizide, die diese neuen Nitromethylen-Derivate als Wirkstoffe enthalten.

Insbesondere betrifft die vorliegende Erfindung neue Nitromethylen-Derivate der nachstehenden allgemeinen Formel (I)

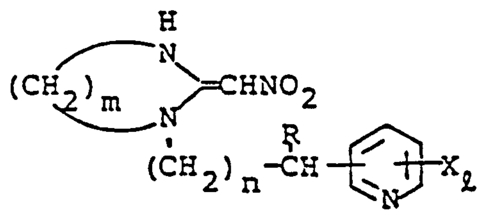

(I)

in welcher

R ein Wasserstoffatom oder eine Alkylgruppe mit 1 bis 4 Kohlenstoffatomen bezeichnet,

X eine Alkylgruppe mit 1 bis 6 Kohlenstoffatomen, eine Alkoxygruppe mit 1 bis 6 Kohlenstoffatomen, eine Alkylthiogruppe mit 1 bis 6 Kohlenstoffatomen, eine Alkylsulfinylgruppe mit 1 bis 6 Kohlenstoffatomen, eine Alkylsulfonylgruppe mit 1 bis 6 Kohlenstoffatomen, eine Alkenylgruppe mit 2 bis 6 Kohlenstoffatomen, eine Alkinylgruppe mit 2 bis 4 Kohlensloffatomen, Phenyl, α-Naphthyl, β-Naphthyl, gegebenenfalls durch Halogen substituiertes Phenoxy, gegebenenfalls durch Halogen substituiertes α-Naphthoxy, gegebenenfalls durch Halogen substituiertes β-Naphthoxy, gegebenenfalls durch Halogen substituiertes Phenylthio, gegebenenfalls durch Halogen substituiertes α-Naphthylthio, gegebenenfalls durch Halogen substituiertes β-Naphthylthio, Benzyl, Phenylethyl, α-Naphthylmethyl oder Halogen bedeutet.

l für 1, 2, 3 oder 4 steht,

m für 2, 3 oder 4 steht und

n 0, 1, 2 oder 3 bedeutet,

oder ein Salz derselben.

Die Nitromethylen-Derivate der Formel (I) gemäß der vorliegenden Erfindung können mittels des folgenden Verfahrens i) hergestellt werden, auf das sich die vorliegende Erfindung ebenfalls erstreckt.

**Verfahren i:**

Ein Verfahren zur Herstellung der Nitromethylen-Derivate der allgemeinen Formel (I) umfaßt die Reaktion einer Verbindung der allgemeinen Formel

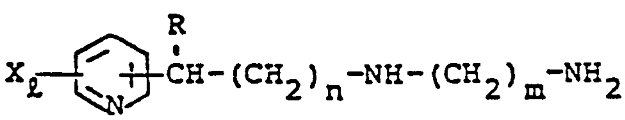

(II)

in der R, X, FIG, m und n die im Vorstehenden angegebenen Bedeutungen haben, mit einer Verbindung der allgemeinen Formel

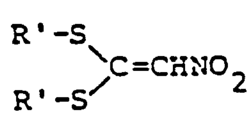

(III)

in der jedes R' eine Alkyl-Gruppe mit 1 bis 6 Kohlenstoffatomen oder eine Benzyl-Gruppe bezeichnet oder die beiden R'-Gruppen zusammen für die Ethylengruppe stehen und mit don ihnen benachbarten Schwefel-Atomen einen Ring bilden können.

Die vorliegende Erfindung betrifft weiterhin Insektizide, die die Nitromethylen-Derivate der allgemeinen Formel (I) als Wirkstoffe enthalten.

Bei der Herstellung der neuen Nitromethylen-Derivate der allgemeinen Formel (I) gemäß der vorliegenden Erfindung sind die Verbindungen der allgemeinen Formel (II), die Zwischenprodukte sind, neue Verbindungen und die vorliegende Erfindung betrifft auch diese Verbindungen (Zwischenprodukte).

Die neuen Verbindungen der allgemeinen Formel (II) können mittels des folgenden Verfahrens ii) hergestellt werden, auf das sich die vorliegende Erfindung ebenfalls erstreckt.

**Verfahren ii:**

Ein Verfahren zur Herstellung der Verbindungen der allgemeinen Formel (II) umfaßt die Reaktion einer Verbindung der allgemeinen Formel

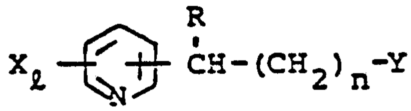 (IV)

in der R, X, FIG und n die im Vorstehenden angegebenen Bedeutungen haben und Y ein Halogen-Atom oder die Gruppe $-OSO_2R''$ bezeichnet, in der R″ für eine niedere Alkyl- oder eine Aryl-Gruppe steht, mit einer Verbindung der allgemeinen Formel

$$H_2N-(CH_2)_m-NH_2 \quad (V) ,$$

in der m die im Vorstehenden angegebene Bedeutung hat.

Verbindungen der allgemeinen Formel (IV) sind entweder bekannt oder können mittels bekannter Herstellungsverfahren hergestellt werden.

Aus der DE-OS-2 514 402 ist bekannt, daß 2-Nitromethylen-imidazolin-Derivate und 2-Nitromethylenhexahydropyrimidin-Derivate der nachstehenden allgemeinen Formel

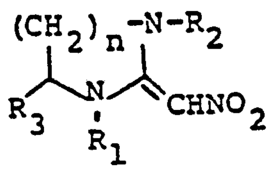

insektizide Aktivität zeigen. Die vorstehende allgemeine Formel umfaßt Fälle mit n = 2, $R_1$ = Phenyl-($C_1$-$C_2$)-alkyl-Gruppe und $R_2$ = $R_3$ = Wasserstoff, und die Beschreibung der DE-OS-2 514 402 beschreibt eine Verbindung der nachstehenden Formel:

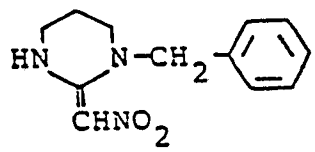 (A-1)

Die DE-OS-2 732 660 erwähnt, daß 1-substituierte Benzyl-2-nitromethylen-imidazolidin-Derivate der nachstehenden allgemeinen Formel

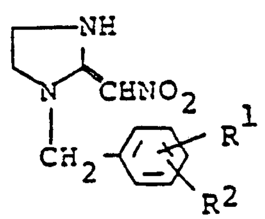

insektizide Wirkung besitzen. Die letztgenannte Deutschen Offenlegungsschrift beschreibt eine Verbindung der nachstehenden Formel:

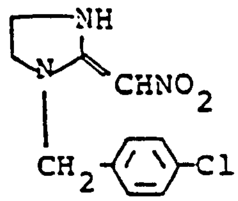

(B-1)

Es wurde gefunden, daß die Verbindungen gemäß der vorliegenden Erfindung bei niedrigerer Dosierung eine hervorragende Bekämpfungswirkung im Vergleich zu den in den oben zitierten Deutschen Offenlegungsschriften beschriebenen Verbindungen (A - 1) und (B - 1), die den Verbindungen gemäß der vorliegenden Erfindung am ähnlichsten sind, besitzen und daß die Verbindungen gemäß der vorliegenden Erfindung eine ausgeprägte Bekämpfungswirkung gegenüber schädlichen Insekten besitzen, die gegen Insektizide des Organophosphat-Typs und Carbamat-Typs aufgrund langandauernder Anwendung letzterer Resistenz entwickelt haben, insbesondere gegenüber saugenden Insekten, wie sie typisch verkörpert werden durch Insekten der Ordnung Hemiptera wie Aphiden, Laternenträger und Zikaden.

Ziel der vorliegenden Erfindung ist es, neue Nitromethylen-Derivate der allgemeinen Formel (I), Zwischenprodukte für diese, Verfahren zu ihrer Herstellung und ihre Verwendung als Insektizide verfügbar zu machen.

Die aktiven Verbindungen gemäß der vorliegenden Erfindung zeigen eine Bekämpfungswirkung gegen schädliche Insekten, ohne daß Phytotoxizität gegenüber Kulturpflanzen auftritt. Des weiteren können die Verbindungen der vorliegenden Erfindung zur Bekämpfung und Ausrottung einer breiten Palette von Schädlingen eingesetzt werden, darunter saugenden Insekten, beißenden Insekten und anderen Pflanzenparasiten, Schädlingen von Lagergetreide und gesundheitsgefährdenden Schädlingen.

Beispiele für diese Schädlinge sind nachstehend aufgeführt:

Insekten der Ordnung Coleoptera

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus.

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella
Plutella maculipennis und
Phyllocnistis citrella.

Insekten der Ordnung Hemiptera

Nephotettix cincticeps,
Nilaparvata lugens,
Pseudococcus comstocki,
Unaspis yanonensis,
Myzus persicae,
Aphis pomi,
Aphis gossypii,

Rhopalosiphum pseudobrassicas,
Stephanitis nashi,
Nazara spp.,
Cimex lectularius,
Trialeurodes vaporariorum und
Psylla spp..

<u>Insekten der Ordnung Orthoptera</u>
Blatella germanica,
Periplaneta americana,
Gryllotalpa africana und
Locusta migratoria migratoriodes.

<u>Insekten der Ordnung Isoptera</u>
Deucotermes speratus und
Coptotermes formosanus.

<u>Insekten der Ordnung Diptera</u>
Musca domestica,
Aedes aegypti,
Hylemia platura,
Culex pipiens,
Anopheles sinensis und
Culex tritaeniorhynchus.

Auf dem Gebiet der Veterinärmedizin sind die neuen Verbindungen gemäß der vorliegenden Erfindung wirksam gegen verschiedene schädliche Tierparasiten (Endo- und Ektoparasiten) wie Insekten und Würmer. Beispiele für solche Tierparasiten sind nachstehend angegeben.

<u>Insekten</u>
Gastrophilus spp.,
Stomoxys spp.,
Trichodectes spp.
Rhodnius spp. und
Ctenocephalidex canis

Substanzen, die eine pestizide Aktivität gegenüber all diesen Schädlingen aufweisen, werden in der vorliegenden Anmeldung gelegentlich einfach als "Insektizide" bezeichnet.

Die Nitromethylen-Derivate der allgemeinen Formel (I) gemäß der vorliegenden Erfindung können in einfacher Weise hergestellt werden, beispielsweise mit Hilfe des folgenden Verfahrens i).

<u>Verfahren i)</u>

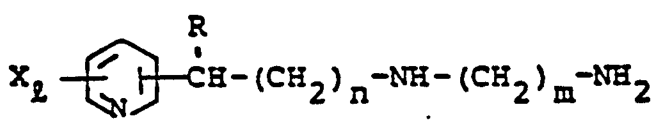

+ (II)

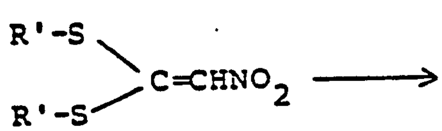

(III)

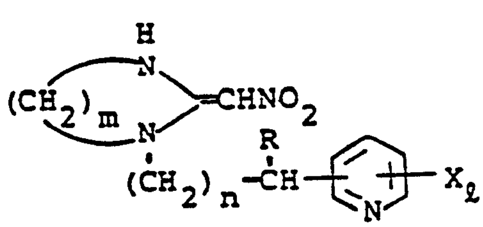

(I)

(In den Formeln haben R, X, FIG, m, n und R' die im Vorstehenden angegebenen Bedeutungen).

In dem vorstehenden Reaktionsschema bezeichnet R ein Wasserstoff-Atom oder eine niedere Alkyl-Gruppe wie zum Beispiel Methyl, Ethyl, Propyl, Isopropyl und n- (iso-, sec- oder tert-) -Butyl.

X bezeichnet die unter Formel (I) angegebenen Bedeutungen.

l bezeichnet 1, 2, 3 oder 4.

m bezeichnet 2, 3 oder 4.

n bezeichnet 0, 1, 2 oder 3.

R' besitzt die unter Formel (III) vorne angegebenen Bedeutungen.

Das vorgenannte Verfahren wird durch das folgende typische Beispiel im einzelnen beschrieben:

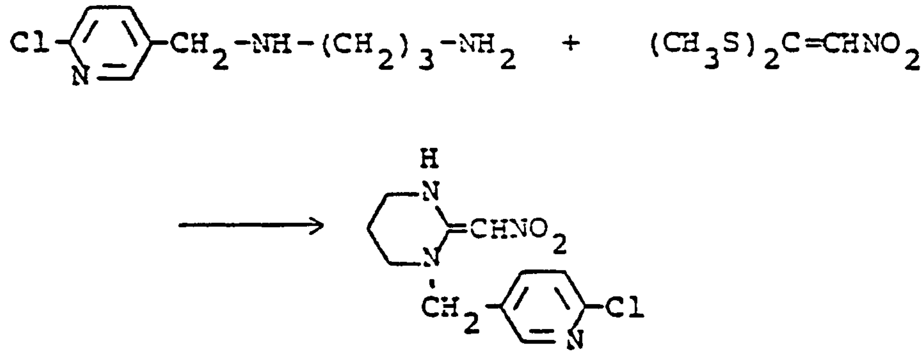

Zweckmäßigerweise kann das vorstehende Verfahren zur Herstellung der Verbindungen gemäß der vorliegenden Erfindung in einem Lösungsmittel oder Verdünnungsmittel durchgeführt werden. Zu diesem Zweck können sämtliche inerten Lösungsmittel oder Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Das obige Verfahren kann innerhalb eines breiten Temperaturbereichs durchgeführt werden. Im allgemeinen kann es bei einer Temperatur zwischen etwa -20°C und dem Siedepunkt der Mischung, vorzugsweise bei einer Temperatur zwischen etwa 0°C und etwa 100°C, durchgeführt werden. Zweckmäßigerweise wird die Reaktion unter normalem Atmosphärendruck durchgeführt, jedoch ist es ebenfalls möglich, bei erhöhtem oder vermindertem Druck zu arbeiten.

Die Verbindungen der allgemeinen Formel (I) gemäß der vorliegenden Erfindung können auch mit Hilfe eines im Folgenden schematisch dargestellten Alternativverfahrens hergestellt werden.

**Alternativ-Verfahren 1**

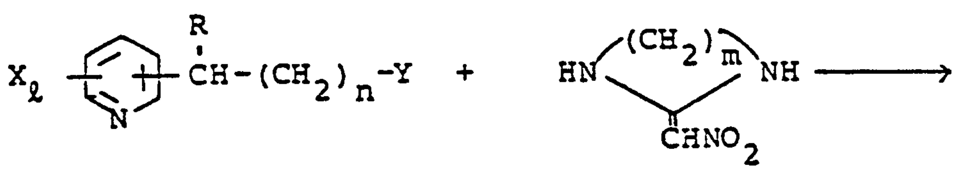

(IV) (VI)

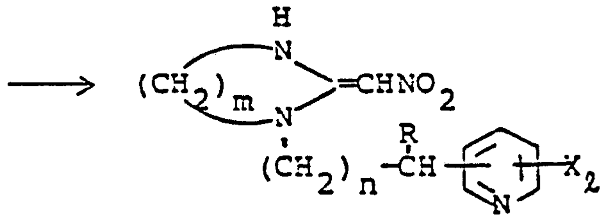

(I)

(In den vorstehenden Formeln haben R, X, FIG, m, n und y die im Vorstehenden angegebenen Bedeutungen).

Die Verbindungen der allgemeinen Formel (II) gemäß der Vorliegenden Erfindung können beispielsweise mit Hilfe des folgenden Verfahrens ii) hergestellt werden.

Verfahren ii)

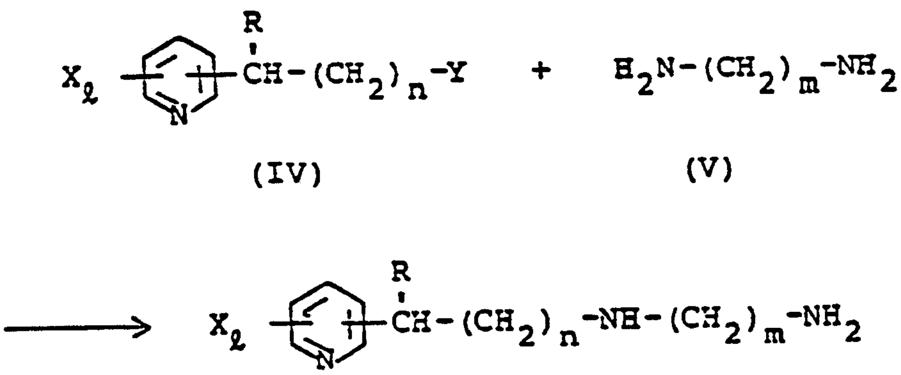

(IV) (V)

(II)

(In den Formeln haben R, X, FIG, m, n und Y die im Vorstehenden angegebenen Bedeutungen).

In dem vorstehenden Reaktionsschema können R, X, FIG, m und n die gleichen Bedeutungen haben, wie sie oben für das Verfahren i) angegeben sind. Y bezeichnet ein Halogen-Atom wie Fluor, Chlor, Brom und Iod oder die Gruppe -$OSO_2R''$, in der R'' für das gleiche Niederalkyl, wie es oben beispielhaft für das Verfahren angegeben ist, oder für eine Aryl-Gruppe wie Phenyl und p-Tolyl steht.

Das vorgenannte Verfahren wird durch das folgende typische Beispiel im einzelnen beschrieben.

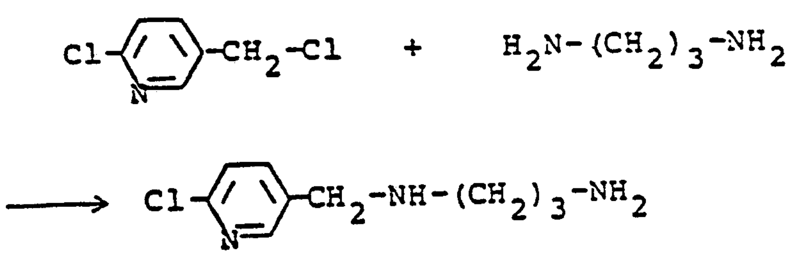

Das vorstehende Verfahren kann unter Verwendung der gleichen Lösungsmittel oder Verdünnungsmittel durchgeführt werden, wie sie im Vorstehenden beispielhaft für das Verfahren i) aufgeführt sind.

Die vorstehende Reaktion kann in Gegenwart eines säurebindenden Mittels durchgeführt werden. Beispiele für das säurebindende Mittel sind die Hydroxide, Carbonate, Hydrogencarbonate und Alkoholate von Alkalimetallen sowie tertiäre Amine wie Triethylamin, Diethylanilin und Pyridin, die im allgemeinen verwendet werden.

Das vorstehende Verfahren kann wie im Fall des Verfahrens i) innerhalb eines weiten Bereichs der Temperatur durchgeführt werden. Die Reaktion wird zweckmäßigerweise unter normalem Atmosphärendruck durchgeführt, jedoch ist es ebenfalls möglich, bei erhöhtem oder vermindertem Druck zu arbeiten.

Die Verbindungen der allgemeinen Formel (II) können, vorausgesetzt, daß n 0 bezeichnet, zusätzlich zu dem vorstehenden Verfahren ii) auch mit Hilfe eines im Folgenden schematisch dargestellten Alternativverfahrens 2 hergestellt werden.

**Alternativ-Verfahren 2**

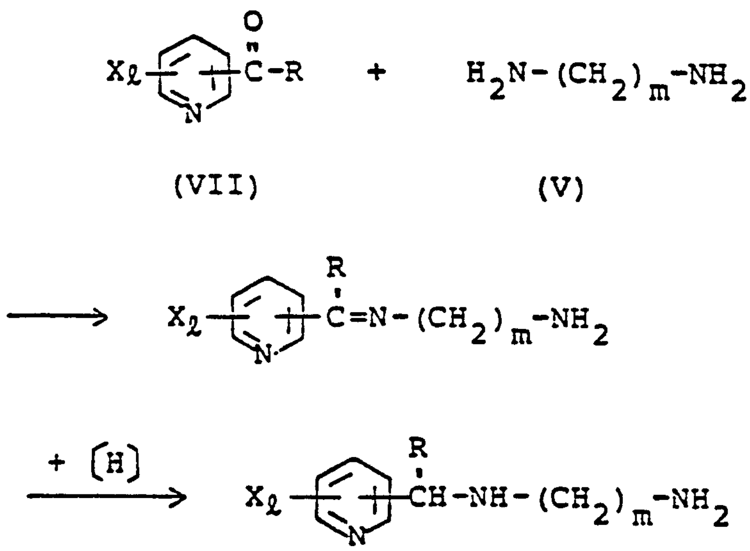

(VII) (V)

(II')

(In den Formeln haben X, R, FIG und m die im Vorstehenden angegebenen Bedeutungen).

Wie das vorstehende Reaktionsschema zeigt, kann die Verbindung der allgemeinen Formel (II') hergestellt werden durch Reaktion eines Reduktionsmittels wie Natriumborhydrid ($NaBH_4$) und dergleichen mit den Iminen, die durch Umsetzung der Pyridinaldehyd-Derivate oder der Pyridylalkylketon-Derivate der allgemeinen Formel (VII) mit der Verbindung der allgemeinen Formel (V) erhalten werden.

Außerdem können die Verbindungen der allgemeinen Formel (II), vorausgesetzt, daß R für ein Wasserstoff-Atom steht und n 0 bezeichnet, auch mit Hilfe des im Folgenden schematisch dargestellten Alternativverfahrens 3 hergestellt werden.

**Alternativ-Verfahren 3**

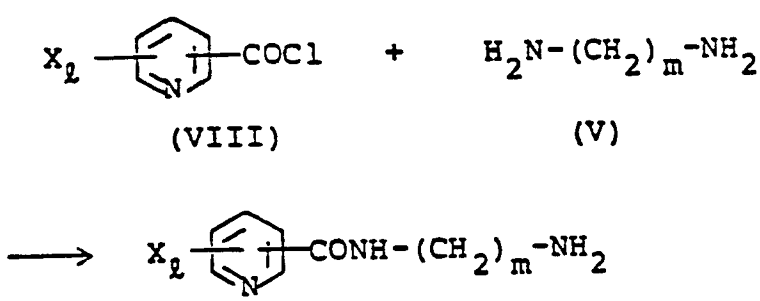

(VIII) (V)

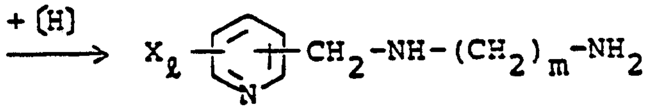


(II")

(In den Formeln haben X, FIG und m die im Vorstehenden angegebenen Bedeutungen).

Wie das vorstehende Reaktionsschema zeigt, kann die Verbindung der allgemeinen Formel (II") hergestellt werden durch Reaktion eines Reduktionsmittels wie Lithiumaluminiumhydrid ($LiAlH_4$) und dergleichen mit den Nicotinsäureamid-Derivaten oder Picolinsäureamid-Derivaten, die durch Umsetzung der Pyridylcarbonyl-Derivate der allgemeinen Formel (VIII) mit der Verbindung der allgemeinen Formel (V) erhalten werden.

Die Verbindungen der allgemeinen Formel (II), vorausgesetzt, daß R für ein Wasserstoff-Atom steht, n 0 bezeichnet und m 3 bezeichnet, können auch mit Hilfe des im Folgenden schematisch dargestellten Alternativverfahrens 4 hergestellt werden.

**Alternativ-Verfahren 4**

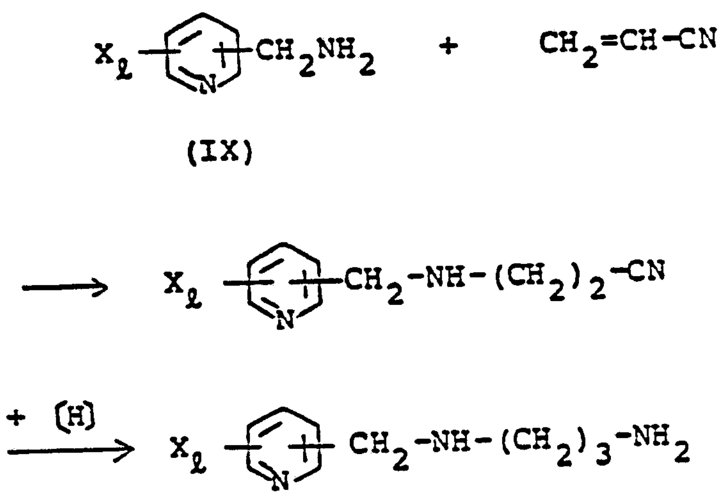


(IX)

(II"')

(In den Formeln haben X und FIG die im Vorstehenden angegebenen Bedeutungen).

Wie das vorstehende Reaktionsschema zeigt, kann die Verbindung der allgemeinen Formel (II"') hergestellt werden durch Reduktion, entsprechend dem oben beschriebenen Alternativverfahren 2, des Addukts, das durch Umsetzung von Acrylnitril mit einer Verbindung der allgemeinen Formel (IX) erhalten wird. Beispielsweise kann N-(2-Chloro-5-pyridylmethyl)trimethylendiamin, die weiter unten genannte Verbindung II - 97, auch mit Hilfe des obigen Alternativverfahrens 4 hergestellt werden.

Die Verbindungen der allgemeinen Formel (II), vorausgesetzt, daß m 2 bezeichnet, können auch mit Hilfe des im Folgenden schematisch dargestellten Alternativverfahrens 5 hergestellt werden.

**Alternativ-Verfahren 5**

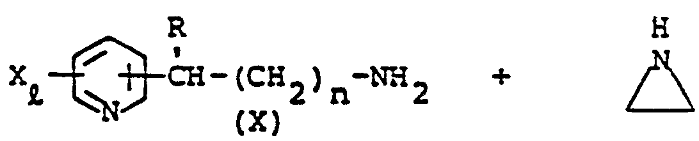

(X)

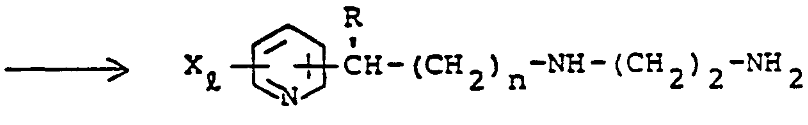

(II'''')

(In den Formeln haben R, X, FIG und n die im Vorstehenden angegebenen Bedeutungen).

Wie das vorstehende Reaktionsschema zeigt, kann die Verbindung der allgemeinen Formel (II'''') auch hergestellt werden durch Reaktion des Pyridylalkylamin-Derivats der allgemeinen Formel (X) mit Ethylenimin.

Als Insektizide können die Verbindungen der vorliegenden Erfindung unmittelbar nach dem Verdünnen mit Wasser oder aber in Form verschiedenartiger Formulierungen zur Anwendung gebracht werden, wie sie unter Verwendug landwirtschaftlich unbedenklicher Hilfsstoffe durch Verfahren gewonnen werden, die bei der praktischen Herstellung von Agrochemikalien allgemein angewandt werden. Im praktischen Gebrauch werden diese verschied. Formulierungen entweder unmittelbar oder nach dem Verdünnen mit Wasser auf die gewünschte Konzentration eingesetzt.

Zu den hierin erwähnten landwirtschaftlich unbedenklichen Hilfsstoffen zählen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel Aerosol-Treibmittel und synergistische Mittel.

Beispiele für die Lösungsmittel sind Wasser und organische Lösungsmittel, beispielweise Kohlenwasserstoffe [z. B. n-Hexan, Petrolether, Erdöl-Fraktionen (z. B. Paraffinwachse, Kerosin, Leichtöle, Mittelöle und Schweröle), Benzol, Toluol un Xylol], halogenierte Kohlenwasserstoffe (z. B. Methylenchlorid, Kohlenstofftetrachlorid, Ethylenchlorid, Ethylendibromid, Chlorbenzol un Chloroform), Alkohole (z. B. Methanol, Ethanol, Propanol und Ethylenglycol), Ether (z. B. Diethylether, Ethylenoxid und Dioxan), Alkoholether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie Getreidepulver, Stärkearten, verarbeitete Stärkearten, Zucker, Glucose uns zerkleinerte Stengel von Pflanzen, sowie Pulver aus syntheitischen Harzen wie Phenol-Harzen, Harnstoff-Harzen und Vinylchlorid-Harzen.

Beispiel für die oberflächenaktiven Mittel umfassen anionische oberflächenaktive Mittel wie Alkylsulfonsäureester (z. B. Natriumlaurylsulfat), Arylsulfonsäuren (z. B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z. B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z. B. Trichlorofluoromethan, Dichlorofluoromethan, 1,2,2-Trichloro-1,1,2-trifluoroethan, Chlorbenzol, verflüssigtes Erdgas (LNG) und niedere Ether), die Verbrennung steuernde Mittel für Räuchermittel (z. B. Nitrite, Zink-Pulver und Dicyandiamid), sauerstoff-abgebende Mittel (z. B. Chlorate), wirkungsverlängernde Mittel, Dispersions-Stabilisatoren [z. B. Casein, Tragant, Carboxymethylcellulose (CMC) und Polyvinylalkohol (PVA)] und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulate, pulvrige Präparate, Räuchermittel, Tabletten, Aerosole, Pasten und Kapseln.

Die Insektizide gemäß der vorliegenden Erfindung können etwa 0,1 bis etwa 95 Gew.-%, vorzugsweise etwa 0,5 bis etwa 90 Gew.-% des vorerwähnten Wirkstoffes enthalten.

Für den praktischen Gebrauch beträgt die geeignete Menge der aktiven Verbindung in den vorgenannten verschiedenartigen Formulierungen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art der Formulierung, dem Verfahren, Zweck, der Zeit und dem Ort seiner Anwendung sowie dem Zustand des Auftretens der schädlichen Insekten variiert werden.

Erforderlichenfalls können die Verbindungen gemäß der vorliegenden Erfindung weiterhin auch in

Kombination mit anderen Agrochemikalien verwendet werden, beispielsweise mit anderen Insektiziden, Fungiziden, anderen Mitiziden, anderen Nematiziden, Anti-Virus-Mitteln, Herbiziden, Pflanzen-Wachstumsregulatoren und Lockstoffen [z. B. Organophosphat-Verbindungen, Carbamat-Verbindungen, Dithio (oder thiol)carbamat-Verbindungen, Organochlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen] und/oder Düngemitteln.

Den im Vorstehenden bezeichneten erfindungsgemäßen Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Spritzen, Bedampfen, Gießen etc.), Oberflächen-Anwendung (z. B. Beschichten, Aufbringen in Form von Bändern, Pulverbeschichten, Bedecken etc.), Eintauchen und Ködern. Sie können auch mittels des sogenannten Ultra-Low-Volume-Sprühverfahrens aufgebracht werden. Nach diesem Verfahren kann der Wirkstoff sogar in einer Konzentration von 100 % eingearbeitet sein.

Die Aufwandmenge pro Flächeneinheit beträgt beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein insektizides Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung schädlicher Insekten verfügbar, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf schädliche Insekten und/oder deren Lebensraum oder den Ort ihres Auftretens aufgebracht wird.

Die vorliegende Erfindung wird durch die folgenden Beispiele im einzelnen erläutert, ist jedoch nicht auf diese speziellen Beispiele allein beschränkt.

**Beispiel 1**

2-Chloro-5-pyridylmethylchlorid (16,2 g) in Form einer Lösung in Acetonitril (30 ml) wurde im Laufe von 1 h tropfenweise bei Raumtemperatur zu einer Lösung von Ethylendiamin (18 g) in Acetonitril (100 ml) hinzugefügt. Die Reaktionsmischung wurde 1 h bei Raumtemperatur und danach 2 h bei 40°C gerührt. Nach dem Rühren wurde das Acetonitril unter vermindertem Druck abgedampft. Dem Rückstand wurde Ether zugesetzt, und die Unlöslichen Salze wurden abfiltriert. Anschließend wurden der Ether und das überschüssige Ethylendiamin unter vermindertem Druck abgedampft, wonach N-(2-Chloro-5-pyridylmethyl)ethylendiamin (16 g) der nachstehenden Formel als farbloses Öl erhalten wurde; $n_D^{20}$ 1,5627.

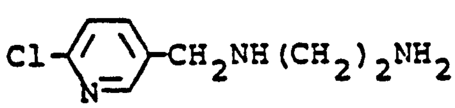

(Verbindung Nr. II - 95)

**Beispiel 2**

2-Bromo-5-pyridylmethylbromid (25 g) in Form einer Lösung in Acetonitril wurde tropfenweise bei 0°C zu einer Lösung von Ethylendiamin (30 g) in Acetonitril (80 ml) hinzugefügt. Nachdem die Reaktionsmischung einige Zeit bei Raumtemperatur gerührt worden war, wurden die gebildeten unlöslichen Salze abfiltriert, und das Filtrat wurde auf dem Wasserbad bei 40°C konzentriert, wonach N-(2-Bromo-5-pyridylmethyl)ethylendiamin (22 g) erhalten wurde; $n_D^{26}$ 1,5586.

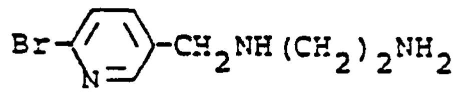

(Verbindung Nr. II - 30)

**Beispiel 3**

6-Methylpicolin-aldehyd (12,1 g) wurde zu einer Lösung von Ethylendiamin (24 g) in wasserfreiem Dioxan (200 ml) hinzugefügt. Die Mischung wurde 3 h bei Raumtemperatur gerührt. Nach dem Rühren wurde die Mischung erhitzt, und ein Gemisch (120 ml) aus Dioxan und entstandenem Wasser wurde zur Vervollständigung der Umsetzung zur Schiff'schen Base abdestilliert. Der Gefäßinhalt wurde auf Raumtemperatur abgekühlt, und Natriumborhydrid (7,6 g) wurde in kleinen Portionen dazugegeben. Nach der Zugabe wurde die Mischung 8 h bei Raumtemperatur gerührt. Nach dem Abdestillieren der flüchtigen Bestandteile aus der Mischung im Vakuum wurde dem Rückstand Eiswasser zugesetzt, und das Produkt wurde mit Chloroform extrahiert. Der Extrakt wurde eingedampft, wonach N-(2-Methyl-6-pyridylmethyl)ethylendiamin (8,3 g) erhalten wurde; Sdp. 127 - 129° C/0,13 mbar (0,1 mm-Hg).

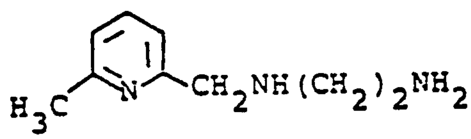

(Verbindung Nr. II - 19)

**Beispiel 4**

2-Ethoxy-5-pyridylmethylchlorid-hydrochlorid (9,6 g) in Form einer wäßrigen Lösung wurde tropfenweise bei 0° C bis 5° C zu der gemischten Lösung von Trimethylendiamin (11,1 g) und einer 20-proz. wäßrigen Lösung (22 g) von Natriumhydroxid hinzugefügt. Nachdem die Reaktionsmischung einige Zeit bei Raumtemperatur gerührt worden war, wurden Wasser und überschüssiges Trimethylendiamin unter vermindertem Druck von dem Gefäßinhalt abdestilliert, und nach dem Abfiltrieren der entstandenen anorganischen Salze wurde die erhaltene viskose ölige Substanz im Vakuum destilliert, wonach N-(2-Ethoxy-5-pyridylmethyl)trimethylendiamin (6,3 g) erhalten wurde; Sdp. 134 - 135° C/0,11 mbar (0,08 mm-Hg).

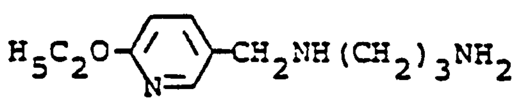

(Verbindung Nr. II - 52)

**Beispiel 5**

Die nachstehende Tabelle 1 zeigt die in gleicher Weise wie in den Beispielen 1, 2 oder 4 hergestellten Verbindungen der allgemeinen Formel (II).

**Tabelle 1**

| Verbindung Nr. | m | n | R | Position d. Subst. am Pyridin | $X_\ell$ | Physikalische Konstante |
|---|---|---|---|---|---|---|
| II - 1 | 2 | 0 | H | 2- | 5-$CH_3$ | $n_D^{20}$ 1,5397 |
| II - 2 | 3 | 0 | H | 2- | 5-$CH_3$ | $n_D^{20}$ 1,5348 |
| II - 20 | 3 | 0 | H | 5- | 2-$CH_3$ | Sdp. 134-135°C/0,13 mbar |
| II - 24 | 2 | 0 | H | 3- | 2-Cl | $n_D^{25}$ 1,5575 |
| II - 31 | 3 | 0 | H | 5- | 2-Br | $n_D^{26}$ 1,5581 |
| II - 33 | 2 | 0 | H | 5- | 2-F | Sdp. 118-120°C/0,13 mbar |
| II - 34 | 3 | 0 | H | 5- | 2-F | Sdp. 120-122°C/0,13 mbar |
| II - 51 | 2 | 0 | H | 5- | 2-$OCH_3$ | Sdp. 142-144°C/0,13 mbar |
| II - 54 | 2 | 0 | H | 5- | 2-O-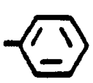 | $n_D^{27}$ 1,5845 |
| II - 55 | 3 | 0 | H | 5- | 2-O-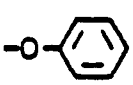 | $n_D^{27}$ 1,5775 |
| II - 58 | 2 | 0 | H | 5- | 2-$SCH_3$ | Sdp. 130-131°C/0,09 mbar |
| II - 59 | 3 | 0 | H | 5- | 2-$SCH_3$ | Sdp. 143-145°C/0,13 mbar |
| II - 97 | 3 | 0 | H | 5- | 2-Cl | $n_D^{22}$ 1,5562 |

Die nachstehenden Bezugsbeispiele erläutern die Synthesen der Verbindungen der allgemeinen Formel (IV), die Zwischenprodukte bei der Herstellung der vorgenannten Verbindungen der allgemeinen Formel (II) sind und von denen ein Teil neu ist.

**Bezugsbeispiel 1a**

15-proz. Methylmercaptan-Natriumsalz (77 g) wurde zu 6-Chloronicotinsäure (15,8 g) gelöst in 20-proz. wäßriger Lösung von Natriumhydroxid (22 g) hinzugegeben. Die Reaktionsmischung wurde 10 h bei 70°C bis 80°C gerührt. Nach dem Abkühlen wurde der nach Neutralisation entstandene Niederschlag durch Filtration gesammelt und aus Chloroform umkristallisiert, wonach 6-Methylthionicotinsäure (15,3 g) erhalten wurde; Schmp. 186 - 188°C.

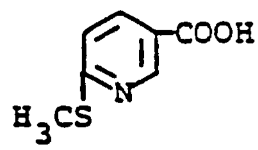

**Bezugsbeispiel 1b**

Thionylchlorid (23,8 g) wurde zu 6-Methylthionicotinsäure (15,3 g) (aus dem vorstehenden Bezugsbeispiel 1a) hinzugefügt. Die Reaktionsmischung wurde allmählich unter Rühren erhitzt und dann unter Rückfluß gehalten, bis die Entwicklung von Hydrogenchlorid beendet war. 6-Methylthionicotinoylchlorid wurde in stöchiometrischer Menge durch Abdestillieren des überschüssigen Thionylchlorids unter vermindertem Druck erhalten. In Ether gelöstes 6-Methylthionicotinoylchlorid wurde tropfenweise bei 0°C bis 10°C zu einer 20-proz. wäßrigen Lösung von Natriumborhydrid (6.5 g) hinzugefügt. Nach der Zugabe wurde die Mischung 1 h gerührt, und die Ether-Schicht wurde abgetrennt und getrocknet. Die Ether-Schicht wurde unter vermindertem Druck eingedampft, wonach 2-Methylthiopyridin-5-methanol (9,6 g) erhalten wurde; $n_D^{22}$ 1,6084.

**Bezugsbeispiel 1c**

Thionylchlorid (7,7 g) wurde zu 2-Methylthiopyridin-5-methanol (7,8 g) (aus dem vorstehenden Bezugsbeispiel 1b) in Chloroform (30 ml) bei Raumtemperatur hinzugefügt. Nachdem einige Zeit gerührt worden war, wurden die flüchtigen Substanzen unter vermindertem Druck abdestilliert, wonach 2-Methylthio-5-chloromethyl-pyridin-hydrochlorid (10,4 g) in stöchiometrischer Menge erhalten wurde; Schmp. 127 - 130°C.

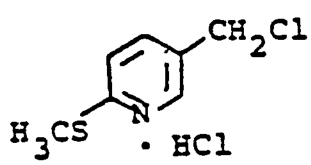

Die auf die gleiche Weise wie in den vorstehenden Bezugsbeispielen 1a, 1b und 1c hergestellten Verbindungen sind im Folgenden beispielhaft aufgeführt.

Verfahren des Bezugsbeispiels 1a:

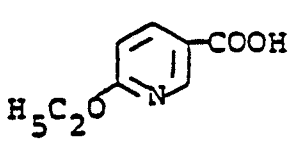

(Schmp. 187 - 190°C)

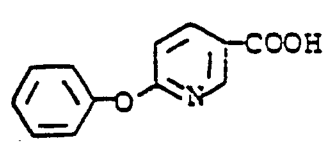

(Schmp. 167 - 168°C)

Verfahren des Bezugsbeispiels 1b:

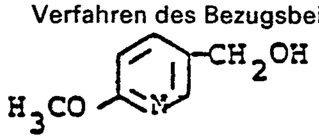

(Sdp. 95 - 96°C/0,67 mbar (0,5 mm-Hg))

(synthetisiert aus 6-Methoxy-nicotinsäure, einer bekannten Verbindung)

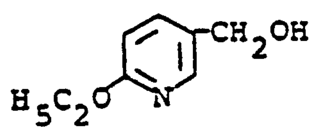

(Sdp. 97 -98° C/0,53 mbar (0,4 mm-Hg))

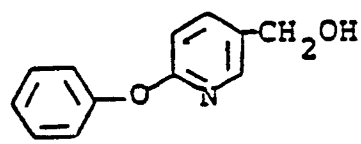

($n_D^{25}$ 1,5960)

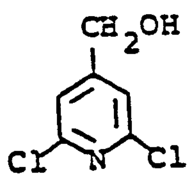

(synthetisiert aus 2,6-Dichloroisonicotinsäure, einer bekannten Verbindung)

(Schmp. 135 - 136° C)

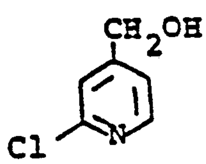

(synthetisiert aus 2-Chloroisonicotinsäure, einer bekannten Verbindung)

(Schmp. 64 - 66° C)

Verfahren des Bezugsbeispiels 1c:

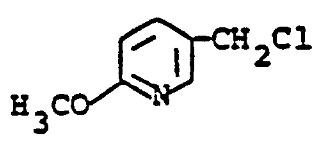

(Sdp. 98 - 99° C/6,7 bis 8 mbar (5 bis 6 mm-Hg))

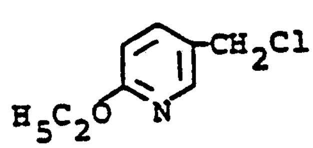

(Sdp. 68 - 70° C/1,33 mbar (1 mm-Hg))

($n_D^{27}$ 1,6088)

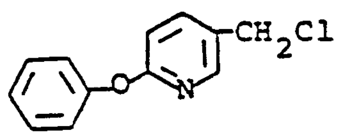

(Schmp. 52 - 53° C)

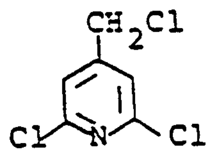

(Sdp. 129 - 130° C/40 mbar (30 mm-Hg))

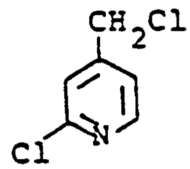

**Beispiel 7**

N-(2-Chloro-5-pyridylmethyl)ethylendiamin (18,6 g), 1-Nitro-2,2-bis(methylthio)ethylen (16,5 g) und Methanol (100 ml) wurden bei Raumtemperatur miteinander vermischt. Die Mischung wurde allmählich unter Rühren erhitzt und weiter bei 50° C gerührt, bis die Entwicklung von Methylmercaptan beendet war. Nach der Reaktion wurde die Reaktionsmischung auf Raumtemperatur abgekühlt, und die erhaltenen Kristalle wurden durch Filtration geammelt, wonach 1-(2-Chloro-5-pyridylmethyl)-2-(nitromethylen)imidazolin (19 g) der nachstehenden Formel in Form blaßgelber Kristalle erhalten wurde; Schmp. 165 - 166° C.

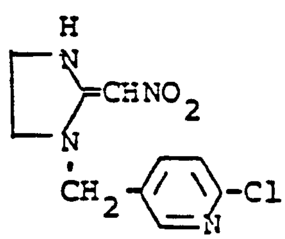

(Verbindung Nr. 95)

**Beispiel 8**

N-(2-Fluoro-5-pyridylmethyl)ethylendiamin (1,7 g), 1-Nitro-2,2-bis(methylthio)ethylen (1,7 g) und Ethanol (10 ml) wurden bei Raumtemperatur miteinander vermischt. Die Mischung wurde allmählich unter Rühren erhitzt und weiter unter Rückfluß gehalten, bis die Entwicklung von Methylmercaptan beendet war. Nach dem Abkühlen auf Raumtemperatur wurden die erhaltenen Kristalle durch Filtration gesammelt und getrocknet, wonach hellgelbe Kristalle von 1-(2-Fluoro-5-pyridylmethyl)-2-(nitromethylen)imidazolin (1,7 g) erhalten wurden; Schmp. 139 - 142° C.

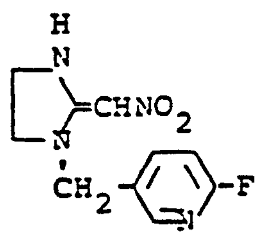

(Verbindung Nr. 1)

**Beispiel 9**

Die nachstehende Tabelle 2 zeigt die in gleicher Weise wie in den Beispielen 7 und 8 hergestellten Verbindungen der allgemeinen Formel (I).

**Tabelle 2**

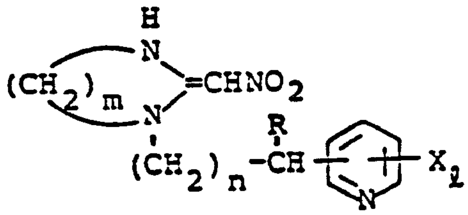

| Verbindung Nr. | m | n | R | Position d. Subst. am Pyridin | $X_\ell$ | Physikalische Konstante |
|---|---|---|---|---|---|---|
| 2 | 2 | 0 | H | 2- | 5-$CH_3$ | Schmp. 130-132°C |
| 3 | 3 | 0 | H | 2- | 5-$CH_3$ | Schmp. 145-146°C |
| 4 | 2 | 0 | H | 2- | 6-Br | Schmp. 176-178°C |
| 5 | 2 | 0 | H | 5- | 2-$OC_2H_5$ | Schmp. 171-173°C |
| 6 | 3 | 0 | H | 5- | 2-$OC_2H_5$ | Schmp. 177-179°C |
| 7 | 2 | 0 | H | 5- | 2-O-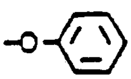 | Schmp. 179-180°C |
| 8 | 3 | 0 | H | 5- | 2-O-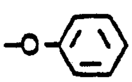 | Schmp. 185-187°C |
| 9 | 3 | 0 | H | 5- | 2-F | Schmp. 149-151°C |

**Tabelle 2** - Fortsetzung ⟶

**Tabelle 2** - Fortsetzung

| Verbindung Nr. | m | n | R | Position d. Subst. am Pyridin | $X_{\ell}$ | Physikalische Konstante |
|---|---|---|---|---|---|---|
| 10 | 2 | 0 | H | 3- | 2-Cl | Schmp. 201-204°C |
| 11 | 2 | 0 | H | 3- | 5-Br | Schmp. 180-182°C |
| 12 | 3 | 0 | H | 3- | 5-Br | Schmp. 192-195°C |
| 13 | 2 | 0 | H | 5- | 2-Br | Schmp. 167-170°C |
| 14 | 3 | 0 | H | 5- | 2-Br | Schmp. 193-195°C |
| 15 | 2 | 0 | H | 4- | 2-Cl | Schmp. 177-179°C |
| 16 | 3 | 0 | H | 4- | 2-Cl | Schmp. 219-222°C |
| 17 | 2 | 0 | H | 4- | 2,6-$Cl_2$ | Schmp. 258-260°C |
| 18 | 2 | 0 | H | 2- | 6-$CH_3$ | Schmp. 191-194°C |
| 19 | 3 | 0 | H | 2- | 6-$CH_3$ | Schmp. 202-204°C |
| 20 | 2 | 0 | H | 2- | 5-Cl | Schmp. 145-147°C |
| 21 | 3 | 0 | H | 2- | 5-Cl | Schmp. 161-162°C |
| 22 | 2 | 0 | H | 5- | 2-$CH_3$ | Schmp. 120-124°C |
| 23 | 3 | 0 | H | 5- | 2-$CH_3$ | Schmp. 138-140°C |
| 24 | 2 | 0 | H | 5- | 2-$SCH_3$ | Schmp. 125-126°C |
| 25 | 3 | 0 | H | 5- | 2-$SCH_3$ | Schmp. 137-139°C |
| 26 | 2 | 0 | H | 4- | 2-Cl 6-$CH_3$ | Schmp. 228-230°C |
| 29 | 2 | 0 | H | 5- | 2-$OCH_3$ | Schmp. 159-161°C |
| 42 | 2 | 0 | H | 5- | 2- 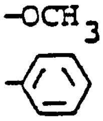 | Schmp. 196-198°C |
| 97 | 3 | 0 | H | 5- | 2-Cl | Schmp. 184-186°C |

**Beispiel 10**

(Benetzbares Pulver)

15 Teile der Verbindung Nr. 95 der Erfindung, 80 Teile eines Gemisches (1 : 5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf schädliche Insekten und/oder ihren Lebensraum oder den Ort ihres Auftretens aufgesprüht.

**Beispiel 11**

(Emulgierbares Konzentrat)

30 Teile der Verbindung Nr. 13 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf schädliche Insekten und/oder ihren Lebensraum oder den Ort ihres Auftretens aufgesprüht.

**Beispiel 12**

(Stäubemittel)

2 Teile der Verbindung Nr. 5 der Erfindung und 98 Teile Tonpulver werden pulverisiert und gemischt, wodurch ein Stäubemittel hergestellt wird. Dieses wird über schädlichen Insekten und/oder ihrem Lebensraum oder dem Ort ihres Auftretens ausgestreut.

**Beispiel 13**

(Granulat)

Wasser (25 Teile) wird zu einer Mischung aus 10 Teilen der Verbindung Nr. 14 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird, wodurch ein Granulat gebildet wird. Das Granulat wird über schädlichen Insekten und/oder ihrem Lebensraum oder dem Ort ihres Auftretens ausgestreut.

**Beispiel 14**

(Granulat)

Ein Drehmischer wird mit 95 Teilen Tonmineral-Teilchen mit einer Teilchengrößen-Verteilung zwischen 0,2 und 2 mm beschickt, und unter Drehen des Mischers werden 5 Teile der Verbindung Nr. 51 der Erfindung auf die Teilchen zur gleichmäßigen Benetzung derselben aufgesprüht. Die feuchte Mischung wird bei 40°C bis 50°C getrocknet, wodurch ein Granulat gebildet wird. Das Granulat wird über schädlichen Insekten und/oder ihrem Lebensraum oder dem Ort ihres Auftretens ausgestreut.

**Beispiel 15**

(Öl-Präparat)

Die Verbindung Nr. 1 der Erfindung (0,5 Teile) und 99,5 Teile Kerosin werden unter Rühren miteinander vermischt, wodurch ein Öl-Präparat gebildet wird. Dieses wird auf schädliche Insekten, Milben oder Nematoden und/oder ihren Lebensraum oder den Ort ihres Auftretens aufgesprüht.

**Beispiel 16**

(Biologischer Test)

Test mit gegen Organophosphor-Mittel resistenten Nephcotettix cincticeps:

Herstellung eines Test-Präparats:

Lösungsmittel: Xylol 3 Gew.-Teile

Emulgator: Polyoxyethylenalkyphenylether 1 Gew.-Teil

Zur Herstellung eines geeigneten Präparats wurde 1 Gew.-Teil der aktiven Verbindung mit der oben bezeichneten Menge Lösungsmittel, das die oben angegebene Menge Emulgator enthielt, vermischt. Die Mischung wurde mit Wasser auf eine vorher festgesetzte Konzentration verdünnt.

Test-Verfahren:

Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Wirkstoff-Konzentration aufweisenden Lösungen jeder der wirksamen Verbindungen, die wie oben angegeben hergestellt wurden, gesprüht. Die aufgesprühte Chemikalie wurde trocknen gelassen, und über die Reispflanzen wurde ein Drahtkorb von 7 cm Durchmesser und 14 cm Höhe gestülpt, unter dem 30 ausgewachsene weibliche Exemplare von Nephotettix cincticeps, die gegen Organophosphor-Mittel resistent waren, ausgesetzt wurden. Die Töpfe wurden jeweils in einem Raum mit konstanter Temperatur aufbewahrt, und 2 Tage später wurde die Zahl der toten Insekten bestimmt und das Vernichtungsverhältnis berechnet.

Die Ergebnisse sind in Tabelle 3 aufgeführt.

**Tabelle 3**

| Verbindung Nr. | Wirkstoff-Konzentration (ppm) | Vernichtungs-verhältnis (%) |
|---|---|---|
| 1 | 8 | 100 |
| 9 | 8 | 100 |
| 13 | 8 | 100 |
| 14 | 8 | 100 |
| 22 | 8 | 100 |
| 24 | 8 | 100 |
| 29 | 8 | 100 |
| 95 | 8 | 100 |
| 97 | 8 | 100 |
| Vergleich | | |
| A - 1 | 40 | 65 |
| B - 1 | 40 | 55 |

Anmerkung:

1) Die Verbindungs-Nummern sind die gleichen, wie sie im Vorstehenden angegeben sind.
2) Die Vergleichs-Verbindungen A - 1 und B - 1 sind die enstprechenden, oben beschriebenen Verbindungen mit den nachstehenden Formeln:

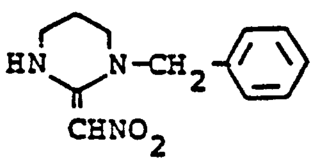

(A-1)

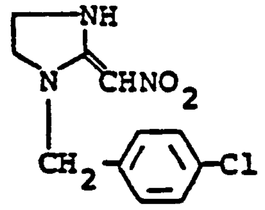

(B-1)

Außer den in Beispiel 17 beispielhaft genannten Verbindungen zeigten beispielsweise die Verbindungen Nr. 48, 49, 50, 51, 55, 96 und 98 ebenfalls ausgezeichnete insektizide Wirkungen in dem gleichen Test wie in Beispiel 17.

**Beispiel 17**

(Biologischer Test)

Test mit Laternenträgern

Test-Verfahren:

Eine wie in Beispiel 17 hergestellte wäßrige Verdünnung mit vorher festgelegter Konzentration der aktiven Verbindung wurde auf etwa 10 cm hohe Reispflanzen, die in Töpfen mit einem Durchmesser von 12 cm gezogen wurden, in einer Menge von 10 ml pro Topf aufgesprüht. Die aufgesprühte Chemikalie wurde trocknen gelassen, und über die Reispflanzen wurde ein Drahtkorb von 7 cm Durchmesser und 14 cm Höhe gestülpt. 30 ausgewachsene weibliche Exemplare von Nilaparvata lugens Stal. eines Stammes, der Resistenz gegen Organophosphor-Mittel zeigte, wurden unter dem Drahtkorb ausgesetzt. Die Töpfe wurden in einem Raum mit konstanter Temperatur aufbewahrt, und 2 Tage später wurde die Zahl der toten Insekten bestimmt. Das Vernichtungsverhältnis wurde danach berechnet.
In gleicher Weise wurde das Vernichtungsverhältnis an Sogatella furcifera Horvath und Organophosphorresistenten Laodelphax striatella Fallen berechnet.
Die Ergebnisse sind in Tabelle 4 aufgeführt.

**Tabelle 4**

| Verbindung Nr. | Wirkstoff-Konzentration (ppm) | Vernichtungsverhältnis (%) | | |
|---|---|---|---|---|
| | | N. lugens | L. striatellus | S. furcifera |
| 1 | 40 | 100 | 100 | 100 |
| 7 | 40 | 100 | 100 | 100 |
| 9 | 40 | 100 | 100 | 100 |
| 13 | 40 | 100 | 100 | 100 |
| 14 | 40 | 100 | 100 | 100 |
| 29 | 40 | 100 | 100 | 100 |
| 50 | 40 | 100 | 100 | 100 |
| 95 | 40 | 100 | 100 | 100 |
| 97 | 40 | 100 | 100 | 100 |
| Vergleich | | | | |
| A - 1 | 40 | 50 | 40 | 40 |
| B - 1 | 40 | 30 | 30 | 30 |

Anmerkung:

1) Die Verbindungs-Nummern sind die gleichen, wie sie im Vorstehenden angegeben sind.
2) Die Vergleichs-Verbindungen A - 1 und B - 1 sind die gleichen, wie sie in der Fußnote zu Tabelle 3 bezeichnet sind.

**Beispiel 18**

(Biologischer Test)

Test mit gegen Organophosphor-Chemikalien und Carbamat-Chemikalien resistenten Myzodes persicae (grünen Pfirsichblattläusen):

Test-Verfahren:

Gezüchtete grüne Pfirsichblattläuse, die Resistenz gegen Organophosphor-Chemikalien und Carbamat-Chemikalien zeigten, wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 17 hergestellte

wäßrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28°C stehen gelassen. 24 Stunden nach dem Sprühen wurde das Vernichtungsverhältnis berechnet. Für jede Verbindung wurde der Test als Doppelbestimmung durchgeführt.

Die Ergebnisse sind in Tabelle 5 aufgeführt.

**Tabelle 5**

| Verbindung Nr. | Wirkstoff-Konzentration (ppm) | Vernichtungs-verhältnis (%) |
|---|---|---|
| 1 | 200 | 100 |
| 9 | 200 | 100 |
| 13 | 200 | 100 |
| 14 | 200 | 100 |
| 25 | 200 | 100 |
| 30 | 200 | 100 |
| 95 | 200 | 100 |
| 97 | 200 | 100 |
| Vergleich | | |
| A - 1 | 1000 | 80 |
| | 200 | 30 |
| B - 1 | 1000 | 60 |
| | 200 | 10 |
| Estox | 1000 | 100 |
| (Handelsprodukt) | 200 | 20 |

Anmerkung:

1) Die Verbindungs-Nummern und die Vergleichs-Verbindungen A - 1 und B - 1 sind die gleichen, wie sie im Vorstehenden angegeben sind.
2) Estox: S-2-Ethylsulfinyl-1-methylethyldimethyl-phosphorothiolat (45 % emulgierbares Konzentrat).

## Patentansprüche

1. Nitromethylen-Derivat der allgemeinen Formel

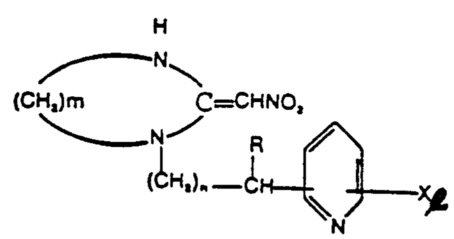 (I)

in welcher

R ein Wasserstoffatom oder eine Alkylgruppe mit 1 bis 4 Kohlenstoffatomen bezeichnet,
X eine Alkylgruppe nit 1 bis 6 Kohlenstoffatomen, eine Alkoxygruppe mit 1 bis 6 Kohlenstoffatomen, eine Alkylthiogruppe mit 1 bis 6 Kohlenstoffatomen, eine Alkylsulfinylgruppe mit 1 bis 6 Kohlenstoffatomen, eine Alkylsulfonylgruppe mit 1 bis 6 Kohlenstoffatomen, eine Alkenylgruppe mit 2 bis 6 Kohlenstoffatomen, eine Alkinylgruppe mit 2 bis 4 Kohlenstoffatomen, Phenyl, α-Haphthyl, β-Naphthyl, gegebenenfalls durch Halogen substituiertes Phenoxy, gegebenenfalls durch Halogen substituiertes α-Naphthoxy, gegebenenfalls durch Halogen substituiertes β-Naphthoxy, gegebenenfalls durch Halogen substituiertes Phenylthio, gegebenenfalls durch Halogen substituiertes α-Naphthylthio, gegebenenfalls durch Halogen substituiertes β-Naphthylthio, Benzyl, Phenylethyl, α-Naphthylmethyl oder Halogen bedeutet,

l für 1, 2, 3 oder 4 steht,
m für 2, 3 oder 4 steht und
n 0, 1, 2 oder 3 bedeutet,

oder ein Salz derselben.

2. Nitromelhylen-Derivat oder Salz desselben nach Anspruch 1, dadurch gekennzeichnet, daß m 2 oder 3 ist.

3. Verfahren zur Herstellung eines Nitromethylen-Derivats der allgemeinen Formel

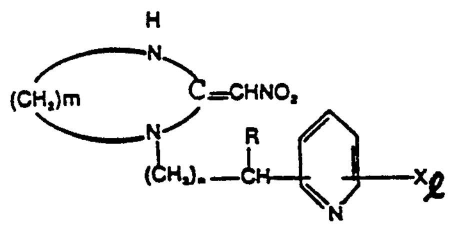

(I)

in welcher

R ein Wasserstoffatom oder eine Alkylgruppe mit 1 bis 4 Kohlenstoffatomen bezeichnet,
X eine Alkylgruppe mit 1 bis 6 Kohlenstoffatomen, eine Alkoxygruppe mit 1 bis 6 Kohlenstoffatomen, eine Alkylthiogruppe mit 1 bis 6 Kohlenstoffatomen, eine Alkylsulfinylgruppe mit 1 bis 6 Kohlenstoffatomen, eine Alkylsulfonylgruppe mit 1 bis 6 Kohlenstoffatomen, eine Alkenylgruppe mit 2 bis 6 Kohlenstoffatomen, eine Alkinylgruppe mit 2 bis 4 Kohlenstoffatomen, Phenyl, α-Naphthyl, β-Naphthyl, gegebenenfalls durch Halogen substituiertes Phenoxy, gegebenenfalls durch Halogen substituiertes α-Naphthoxy, gegebenenfalls durch Halogen substituiertes β-Naphthoxy, gegebenenfalls durch Halogen substituiertes Phenylthio, gegebenenfalls durch Halogen substituiertes α-Naphthylthio, gegebenenfalls durch Halogen substituiertes β-Naphthylthio, Benzyl, Phenylethyl, α-Naphthylmethyl oder Halogen bedeutet,

l für 1, 2, 3 oder 4 steht,
m für 2, 3 oder 4 steht und
n 0, 1, 2 oder 3 bedeutet

oder eines Salzes desselben,
dadurch gekennzeichnet, daß eine Verbindung der allgemeinen Formel (II)

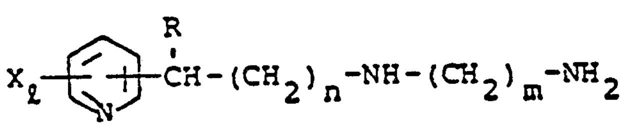

(II)

in der R, X, FIG, m und n die im Vorstehenden angegebenen Bedeutungen haben, mit einer Verbindung der allgemeinen Formel (III)

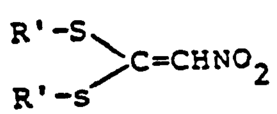

(III)

in der jedes R' eine Alkyl-Gruppe mit 1 bis 6 Kohlenstoffatomen oder eine Benzyl-Gruppe bezeichnet oder die beiden R'-Gruppen zusammen für die Ethylengruppe stehen und mit den ihnen benachbarten Schwefel-Atomen einen Ring bilden können, umgesetzt wird.

4. Insektizides Mittel, gekennzeichnet durch einen Gehalt an mindestens einem Nitromethylen-Derivat der Formel

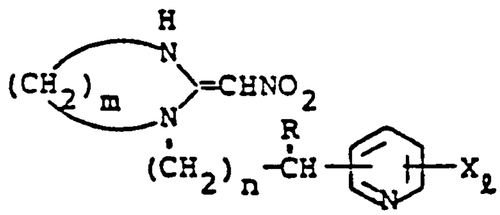

(I)

in der

R ein Wasserstoffatom oder eine Alkylgruppe mit 1 bis 4 Kohlenstoffatomen bezeichnet,
X eine Alkylgruppe mit 1 bis 6 Kohlenstoffatomen, eine Alkoxygruppe mit 1 bis 6 Kohlenstoffatomen, eine Alkylthiogruppe mit 1 bis 6 Kohlenstoffatomen, eine Alkylsulfinylgruppe mit 1 bis 6 Kohlenstoffatomen, eine Alkylsulfonylgruppe mit 1 bis 6 Kohlenstoffatomen, eine Alkenylgruppe mit 2 bis 6 Kohlenstoffatomen, eine Alkinylgruppe mit 2 bis 4 Kohlenstoffatomen, Phenyl, α-Naphthyl, β-Naphthyl, gegebenenfalls durch Halogen substituiertes Phenoxy, gegebenenfalls durch Halogen substituiertes α-Naphthoxy, gegebenenfalls durch Halogen substituiertes β-Naphthoxy, gegebenenfalls durch Halogen substituiertes Phenylthio, gegebenenfalls durch Halogen substituiertes α-Naphthylthio, gegebenenfalls durch Halogen substituiertes β-Naphthylthio, Benzyl, Phenylethyl, α-Naphthylmethyl oder Halogen bedeutet,

l für 1, 2, 3 oder 4 steht,
m für 2, 3 oder 4 steht und
n 0, 1, 2 oder 3 bedeutet,

oder ein Salz desselben.

5. Verbindung der allgemeinen Formel (II)

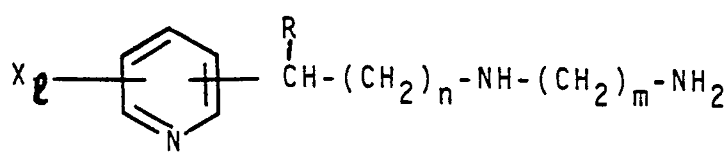

(II)

in der

R ein Wasserstoffatom oder eine Alkylgruppe mit 1 bis 4 Kohlenstoffatomen bezeichnet,
X eine Alkylgruppe mit 1 bis 6 Kohlenstoffatomen, eine Alkoxygruppe mit 1 bis 6 Kohlenstoffatomen, eine Alkylthiogruppe mit 1 bis 6 Kohlenstoffatomen, eine Alkylsulfinylgruppe mit 1 bis 6 Kohlenstoffatomen, eine Alkylsulfonylgruppe mit 1 bis 6 Kohlenstoffatomen, eine Alkenylgruppe mit 2 bis 6 Kohlenstoffatomen, eine Alkinylgruppe mit 2 bis 4 Kohlenstoffatomen, Phenyl, α-Naphthyl, β-Naphthyl, gegebenenfalls durch Halogen substituiertes Phenoxy, gegebenenfalls durch Halogen substituiertes α-Naphthoxy, gegebenenfalls durch Halogen substituiertes β-Naphthoxy, gegebenenfalls durch Halogen substituiertes Phenylthio, gegebenenfalls durch Halogen substituiertes α-Naphthylthio, gegebenenfalls durch Halogen substituiertes β-Naphthylthio, Benzyl, Phenylethyl, α-Naphthylmethyl oder Halogen bedeutet,

l für 1, 2, 3 oder 4 steht,
m für 2, 3 oder 4 steht und
n 0, 1, 2 oder 3 bedeutet.

6. Verfahren zur Herstellung einer Verbindung der allgemeinen Formel (II)

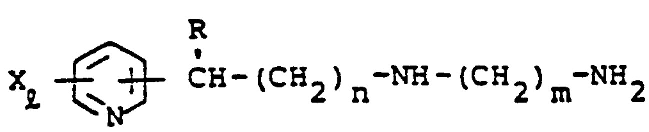

(II)

in welcher
R, X, FIG, m und n die in Anspruch 4 angegebene Bedeutung haben,
dadurch gekennzeichnet, daß eine Verbindung der allgemeinen Formel (IV)

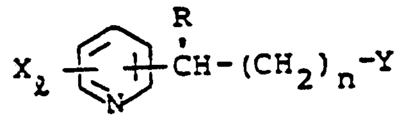

(IV)

in der R, X, FIG und n die im Vorstehenden angegebenen Bedeutungen haben und Y ein Halogen-Atom oder die Gruppe $-OSO_2R''$ bezeichnet, in der R'' für Alkyl ($C_1$-$C_6$) oder eine Phenylgruppe steht, mit einer Verbindung der allgemeinen Formel (V)

$$H_2N-(CH_2)_m-NH_2 \qquad (V)$$

in der m die im Vorstehenden angegebene Bedeutung hat, umgesetzt wird.

7. Verfahren zur Bekämpfung von Insekten, dadurch gekennzeichnet, daß man Nitromethylen-Derivate der Formel (I) auf Insekten oder ihren Lebensraum einwirken läßt.

8. Verwendung von Nitromethylen-Derivaten der Formel (I) zur Bekämpfung von Insekten.

9. Verfahren zur Herstellung von insektiziden Mitteln, dadurch gekennzeichnet, daß man Nitromethylen-Derivate der Formel (I) mit Streckmitteln und/oder oberflächenaktiven Mitteln vermischt.

**Claims**

1. Nitromethylene derivative of the general formula

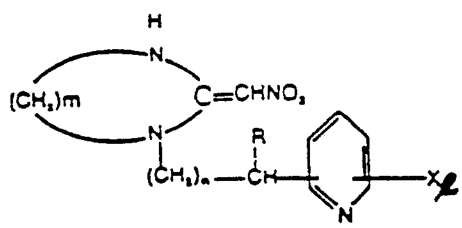

(I)

in which

R denotes a hydrogen atom or an alkyl group having 1 to 4 carbon atoms,

X denotes an alkyl group having 1 to 6 carbon atoms, an alkoxy group having 1 to 6 carbon atoms, an alkylthio group having 1 to 6 carbon atoms an alkylsulphinyl group having 1 to 6 carbon atoms, an alkylsulphonyl group having 1 to 6 carbon atoms, an alkenyl group having 2 to 6 carbon atoms, an alkinyl group having 2 to 4 carbon atoms, phenyl, α-naphthyl, β-naphthyl, phenoxy which is optionally substituted by halogen, α-naphthoxy which is optionally substituted by halogen, β-naphthoxy which is optionally substituled by halogen, phenylthio which is optionally substituted by halogen, α-naphthylthio which is optionally substituted by halogen, β-naphthylthio which is optionally substituted by halogen, or denotes benzyl, phenylethyl, α-naphthylmethyl or halogen,

l stands for 1, 2, 3 or 4,
m stands for 2, 3 or 4 and
n denotes 0, 1, 2 or 3,
or a salt thereof.

2. Nitromethylene derivative or salt thereof according to claim 1, characterized in that m is 2 or 3.

3. Process for the preparation of a nitromethylene derivative of the general formula

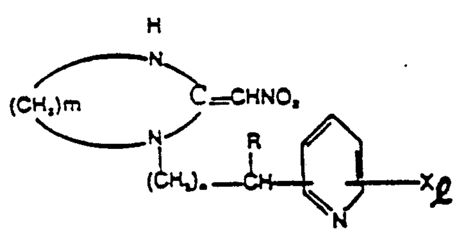

(I)

in which

R denotes a hydrogen atom or an alkyl group having 1 to 4 carbon atoms,

X denotes an alkyl group having 1 to 6 carbon atoms, an alkoxy group having 1 to 6 carbon atoms, an alkylthio group having 1 to 6 carbon atoms, an alkylsulphinyl group having 1 to 6 carbon atoms, an alkylsulphonyl group having 1 to 6 carbon atoms, an alkenyl group having 2 to 6 carbon atoms, an alkinyl group having 2 to 4 carbon atoms, phenyl, α-naphthyl, α-naphthyl, phenoxy which is optionally substituted by halogen, α-naphthoxy which is optionally substituted by halogen, β-naphthoxy which is optionally substituted by halogen, phenylthio which is optionally substituted by halogen, α-naphthylthio which is optionally substituted by halogen, β-naphthylthio which is optionally substituted by halogen, or denotes benzyl, phenylethyl, α-naphthylmethyl or halogen,

l stands for 1, 2, 3 or 4,
m stands for 2, 3 or 4 and
n denotes 0, 1, 2 or 3
or of a salt thereof,
characterized in that a compound of the general formula (II)

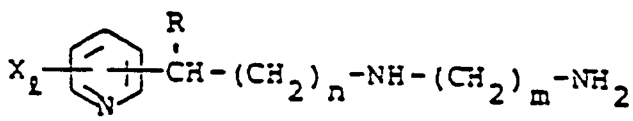

(II)

in which R, X, FIG, m and n have the aforementioned meanings is reacted with a compound of the general formula (III)

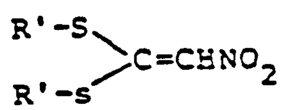

(III)

in which each R' denotes an alkyl group having 1 to 6 carbon atoms or a benzyl group, or the two R' groups together stand for the ethylene group and together with the adjacent sulphur atoms can form a ring.

4. Insecticidal agent, characterized in that it contains at least one nitromethylene derivative of the formula

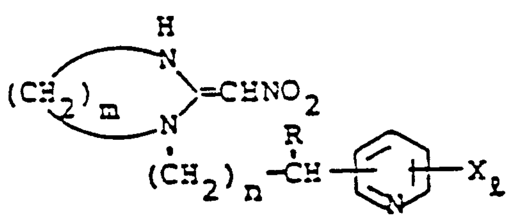

(I)

in which

R denotes a hydrogen atom or an alkyl group having 1 to 4 carbon atoms,
X denotes an alkyl group having 1 to 6 carbon atoms, an alkoxy group having 1 to 6 carbon atoms, an alkylthio group having 1 to 6 carbon atoms, an alkylsulphinyl group having 1 to 6 carbon atoms, an alkylsulphonyl group having 1 to 6 carbon atoms, an alkenyl group having 2 to 6 carbon atoms, an alkinyl group having 2 to 4 carbon atoms, phenyl, α-naphthyl, β-naphthyl, phenoxy which is optionally substituted by halogen, α-naphthoxy which is optionally substituted by halogen, β-naphthoxy which is optionally substituted by halogen, phenylthio which is optionally substituted by halogen, α-naphthylthio which is optionally substituted by halogen, β-naphthylthio which is optionally substituted by halogen, or denotes benzyl, phenylethyl, α-naphthylmethyl or halogen,

l stands for 1, 2, 3 or 4,
m stands for 2, 3 or 4 and
n denotes 0, 1, 2 or 3,
or a salt thereof.

5. Compound of the general formula (II)

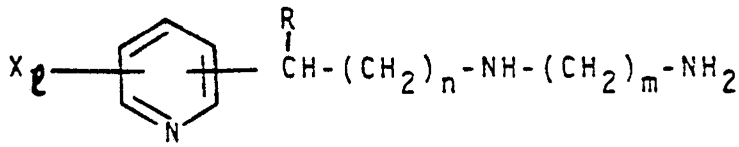

(II)

in which

R denotes a hydrogen atom or an alkyl group having 1 to 4 carbon atoms,

X denotes an alkyl group having 1 to 6 carbon atoms, an alkoxy group having 1 to 6 carbon atoms, an alkylthio group having 1 to 6 carbon atoms, an alkylsulphinyl group having 1 to 6 carbon atoms, an alkylsulphonyl group having 1 to 6 carbon atoms, an alkenyl group having 2 to 6 carbon atoms, an alkinyl group having 2 to 4 carbon atoms, phenyl, α-naphthyl, β-naphthyl, phenoxy which is optionally substituted by halogen, α-naphthoxy which is optionally substituted by halogen, β-naphthoxy which is optionally substituted by halogen, phenylthio which is optionally substituted by halogen, β-naphthylthio which is optionally subsltituted by halogen, or denotes benzyl, phenylethyl, α-naphthylmethyl or halogen,

I stands for 1, 2, 3 or 4,
m stands for 2, 3 or 4 and
n denotes 0, 1, 2 or 3.

6. Process for the preparation of a compound of the general formula (II)

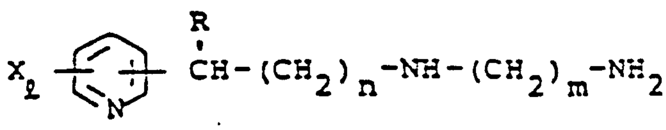

(II)

in which
R, X, FIG, m and n have the meaning given in claim 4,
characterized in that a compound of the general formula (IV)

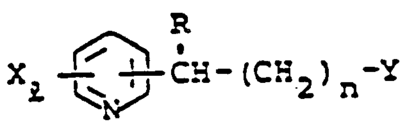

(IV)

in which R, X, FIG and n have the aforementioned meanings and Y denotes a halogen atom or the group $-OSO_2R''$ in which R'' stands for alkyl ($C_1$-$C_6$) or a phenyl group is reacted with a compound of the general formula (V)

$$H_2N-(CH_2)_m-NH_2 \qquad (V)$$

in which m has the aforementioned meaning.

7. Method of controlling insects, characterized in that nitromethylene derivatives of the formula (I) are allowed to act on insects or their habitat.

8. Use of nitromethylene derivatives of the formula (I) for controlling insects.

9. Process for the preparation of insecticidal agents, characterized in that nitromethylene derivatives of the formula (I) are mixed with extenders and/or surface-active agents.

## Revendications

1. Dérivé nitrométhylénique de formule générale

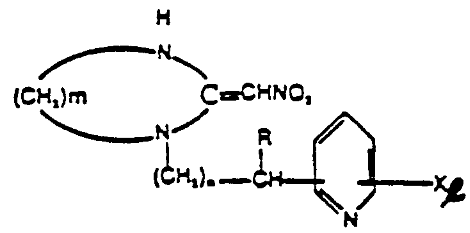

(I)

dans laquelle

R représente un atome d'hydrogène ou un groupe alkyle avec 1 à 4 atomes de carbone,

X représente un groupe alkyle avec 1 à 6 atomes de carbone, un groupe alcoxy avec 1 à 6 atomes de carbone, un groupe alkylthio avec 1 à 6 atomes de carbone, un groupe alkylsulfinyle avec 1 à 6 atomes de carbone un groupe alkylsulfonyle avec 1 à 6 atomes de carbone, un groupe alcényle avec 2 à 6 atomes de carbone, un groupe alcynyle avec 2 à 4 atomes de carbone, phényle, α-naphtyle, β-naphtyle, phénoxy éventuellement substitué par de l'halogène, α-naphtoxy éventuellement substitué par de l'halogène, β-naphtoxy éventuellement substitué par de l'halogène, phénylthio éventuellement substitué par de l'halogène, β-naphtylthio éventuellement substitué par de l'halogène, β-naphtylthio éventuellement substitué par de l'halogène, benzyle, phényléthyle, α-naphtylméthyle ou un halogène,

l signifie 1, 2, 3 ou 4,
m signifie 2, 3 ou 4,
n signifie 0, 1, 2 ou 3,
ou un de ses sels.

2. Dérivé nitrométhylénique ou un de ses sels selon la revendication 1, caractérisé en ce que m signifie 2 ou 3.

3. Procédé pour la fabrication d'un dérivé nitrométhylénique de formule générale

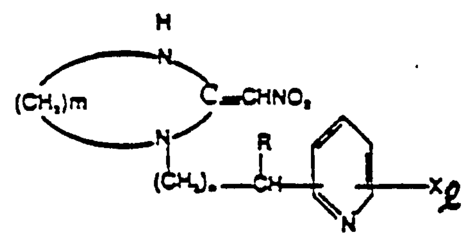

(I)

dans laquelle

R représente un atome d'hydrogène ou un groupe alkyle avec 1 à 4 atomes de carbone,

X représente un groupe alkyle avec 1 à 6 atomes de carbone, un groupe alcoxy avec 1 à 6 atomes de carbone, un groupe alkylthio avec 1 à 6 atomes de carbone, un groupe alkylsulfinyle avec 1 à 6 atomes de carbone, un groupe alkylsulfonyle avec 1 à 6 atomes de carbone, un groupe alcényle avec 2 à 6 atomes de carbone, un groupe alcynyle avec 2 à 4 atomes de carbone, phényle, α-naphtyle, β-naphtyle, phénoxy éventuellement substitué par de l'halogène, α-naphtoxy éventuellement substitué par de l'halogène, β-naphtoxy éventuellement substitué par de l'halogène, phénylthio éventuellement substitué par de l'halogène, α-naphtylthio éventuellement substitué par de l'halogène, β-naphtylthio éventuellement substitué par de l'halogène, benzyle, phényléthyle, α-naphtylméthyle ou un halogène,

l signifie 1, 2, 3 ou 4,
m signifie 2, 3 ou 4,
n signifie 0, 1, 2 ou 3,
ou un de ses sels,
caractérisé en ce qu'on fait réagir un composé de formule générale (II)

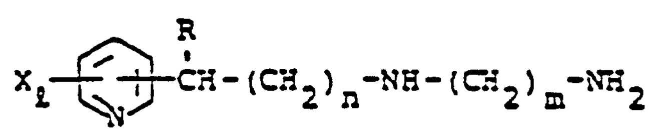

(II)

dans laquelle R, X, FIG, m et n ont les significations décrites ci-dessus, avec un composé de formule générale (III)

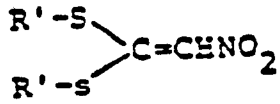

(III)

dans laquelle chaque groupe R' représente un groupe alkyle avec 1 à 6 atomes de carbone ou un groupe benzyle, ou les deux groupes R' représentent ensemble le groupe éthylène et peuvent former un cycle avec les atomes de soufre voisins.

4. Agent insecticide caractérisé en ce qu'il contient au moins un dérivé nitrométhylénique de formule

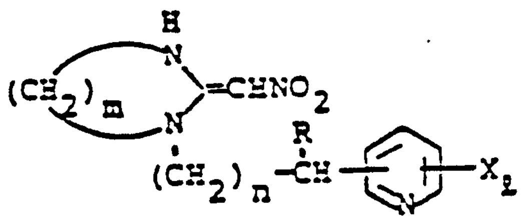

(I)

dans laquelle

R représente un atome d'hydrogène ou un groupe alkyle avec 1 à 4 atomes de carbone,

X représente un groupe alkyle avec 1 à 6 atomes de carbone, un groupe alcoxy avec 1 à 6 atomes de carbone, un groupe alkylthio avec 1 à 6 atomes de carbone, un groupe alkylsulfinyle avec 1 à 6 atomes de carbone, un groupe alkylsulfonyle avec 1 à 6 atomes de carbone, un groupe alcényle avec 2 à 6 atomes de carbone, un groupe alcynyle avec 2 à 4 atomes de carbone, phényle, α-naphtyle, β-naphtyle phénoxy éventuellement substitué par de l'halogène, α-naphtoxy éventuellement substitué par de l'halogène, β-naphtoxy éventuellement substitué par de l'halogène, phénylthio éventuellement substitué par de l'halogène, α-naphtylthio éventuellement substitué par de l'halogène, β-naphtylthio éventuellement substitué par de l'halogène, benzyle, phényléthyle, α-naphtylméthyle ou un halogène,

l signifie 1, 2, 3 ou 4,
m signifie 2, 3 ou 4,
n signifie 0, 1, 2 ou 3
ou un de ses sels.

5. Composé de formule générale (II)

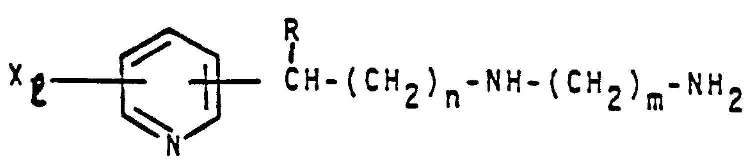

(II)

dans laquelle

R représente un atome d'hydrogène ou un groupe alkyle avec 1 à 4 atomes de carbone,

X représente un groupe alkyle avec 1 à 6 atomes de carbone, un groupe alcoxy avec 1 à 6 atomes de carbone, un groupe alkylthio avec 1 à 6 atomes de carbone, un groupe alkylsulfinyle avec 1 à 6 atomes de carbone, un groupe alkylsulfonyle avec 1 à 6 atomes de carbone, un groupe alcényle avec 2 à 6 atomes de carbone, un groupe alcynyle avec 2 à 4 atomes de carbone, phényle, α-naphtyle, β-naphtyle, phénoxy éventuellement substitué par de l'halogène, α-naphtoxy éventuellement substitué par de l'halogène, β-naphtoxy éventuellement substitué par de l'halogène, phénylthio éventuellement substitué par de l'halogène, α-naphtylthio éventuellement substitué par de l'halogène, β-naphtylthio éventuellement substitué par de l'halogène, benzyle, phényléthyle, α-naphtylméthyle ou un halogène,

l signifie 1, 2, 3 ou 4,
m signifie 2, 3 ou 4,
n signifie 0, 1, 2 ou 3.

6. Procédé pour la fabrication d'un composé de formule générale (II)

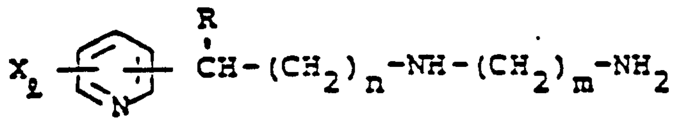

(II)

dans laquelle

R, X, FIG, m et n ont les significations décrites dans la revendication 4, caractérisé en ce qu'on fait réagir un composé de formule générale (IV)

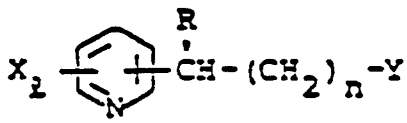

(IV)

dans laquelle R, X, FIG et n ont les significations décrites ci-dessus et Y représente un atome d'halogène ou le groupe $-OSO_2R''$, dans lequel R'' représente un groupe alkyle en ($C_1$-$C_6$) ou un groupe phényle, avec un composé de formule générale (V)

$$H_2N-(CH_2)_m-NH_2 \qquad (V)$$

dans laquelle m a les significations décrites ci-dessus.

7. Procédé pour la lutte contre les insectes, caractérisé en ce que l'on fait agir des dérivés nitrométhyléniques de formule (2) sur des insectes ou leur habitat.

8. Utilisation de dérivés nitrométhyléniques de formule (1) pour la lutte contre les insectes.

9. Procédé pour la fabrication d'agents insecticides, caractérisé en ce que l'on mélange des dérivés nitrométhyléniques de formule (I) avec des diluants et/ou desagents tensio-actifs.